# EUROPÄISCHE PATENTSCHRIFT

(11) **EP 1 868 569 B1**
(45) Veröffentlichungstag und Bekanntmachung des Hinweises auf die Patenterteilung: **04.07.2012**
(21) Anmeldenummer: 06704766.2
(22) Anmeldetag: 24.03.2006
(51) Int. Cl.: A61K 9/08, A61K 9/00, A61K 31/167, A61K 31/135, A61K 31/485, A61K 31/465

(54) **FLÜSSIGE, WÄSSRIGE DARREICHUNGSFORM FÜR SUBLINGUALE ODER NASALE ANWENDUNGEN VON AMINORGRUPPEN AUFWEISENDEN WIRKSTOFFEN**
LIQUID AQUEOUS DOSAGE FORMS FOR SUB-LINGUAL OR NASAL APPLICATIONS OF ACTIVE AGENTS WITH AMINO GROUPS
FORME GALENIQUE AQUEUSE LIQUIDE POUR APPLICATIONS SUBLINGUALES OU NASALES DE PRINCIPES ACTIFS PRESENTANT DES GROUPES AMINO

(30) Priorität: 24.03.2005 AT 5122005
(43) Veröffentlichungstag der Anmeldung: 26.12.2007
(73) Patentinhaber: Globopharm Pharmazeutische Produktions- und Handelsgesellschaft m.b.H., 2700 Wiener Neustadt (AT)
(72) Erfinder: BURGHART, Kurt, A-2700 Wr. Neustadt (AT); BURGHART, Walter, A-1030 Wien (AT)
(74) Vertreter: Miksovsky, Alexander
(86) Internationale Anmeldenummer: PCT/AT2006/000126
(87) Internationale Veröffentlichungsnummer: WO 2006/099651

(56) Entgegenhaltungen:
- EP-A- 0 316 174
- EP-A- 0 407 000
- GB-A- 967 594
- US-A- 5 674 481
- US-B1- 6 767 925
- US-B1- 6 770 263

## Beschreibung

Die Erfindung bezieht sich auf eine flüssige, wässrige Darreichungsform für sublinguale oder nasale Anwendungen von Aminogruppen aufweisenden Wirkstoffen, wie z. B. von Analgetika, insbesondere Opiate, Lokalanästhetika, Antihistaminika oder Nicotin. Unter Aminogruppen werden unsubstituierte Aminogruppen verstanden.

Substanzen der eingangs genannten Art, die sublingual und/oder nasal angewendet werden, kommen in der Regel oral appliziert nicht in voller Wirksamkeit in den Blutkreislauf. Im Fall von Antihistaminika ist es darüber hinaus von wesentlicher Bedeutung, wenn die gewünschte Resorption entsprechend rasch erfolgt, um Chaosreaktionen des Körpers zu verhindern. Ebenso gilt für Analgetika, dass schmerzstillende Mittel nur dann hinreichend rasch zur Wirkung gelangen, wenn die jeweils therapeutisch erforderliche Menge rasch genug resorbiert werden kann. Ebenso wie bei Antihistaminika gilt auch für Pharmazeutika, welche eine abschwellende Wirkung zeigen sollen, dass ein möglichst rascher Wirkungseintritt erforderlich ist.

US6770263, GB967594, EP0407000, EP0316174, US6767925 und US5674481 offenbaren Zusammensetzungen zur sublingualen, nasalen oder buccalen Anwendung von Wirkstoffen, teilweise von solchen mit einer Aminogruppe. Dabei wird Kaliumsorbat als Konservierungsmittel in niedrigen Mengen eingesetzt, höhere wie äquimolare Mengen an Kaliumsorbat liegen nicht vor.

Die Erfindung zielt nun darauf ab, pharmazeutische Wirkstoffe der eingangs genannten Art in einer flüssigen, wässrigen Darreichungsform zur Verfügung zu stellen, welche einen rascheren Wirkungseintritt bei niedrigerer Dosierung oder bei gleicher Dosierung eine längere Wirkungsdauer ermöglicht. Zur Lösung dieser Aufgabe besteht die erfindungsgemäße flüssige, wässrige Darreichungsform im wesentlichen darin, dass der Wirkstoff als pharmakologisch unbedenkliches Salz eingesetzt ist und mit K-Sorbat in wenigstens äquimolarer Menge in der Flüssigkeit vorliegt. Dadurch, dass gemeinsam mit den genannten Wirkstoffen, welche als unbedenkliches Salz, und insbesondere als Hydrochlorid, eingesetzt werden, Kaliumsorbat in der flüssigen, wässrigen Darreichungsform vorliegt, gelingt es, überraschenderweise die Schleimhaut leichter zu überwinden und damit eine raschere Diffusion durch die Mucosa zu ereichen. Analoges gilt für Nasenschleimhäute, wobei in Grenzfällen und besonders bei hohen Konzentrationen Trübungen gebildet werden können, die durch Einsatz von Zusatzstoffen (Lösungsmittel, Lösungsvermittler) wieder beseitigt werden müssen. Abweichend von den üblichen wässrigen Darreichungsformen, bei denen als Wirkstoffe Hydrochloride eingesetzt werden, wird bei Zusatz K-Sorbat beobachtet, dass im neutralen pH-Bereich, d.h. insbesondere im pH-Bereich zwischen 5,0 und 7,5 die flüssigen, wässrigen Darreichungsformen empfindlicher gegen oxidativen Abbau sind. Aus diesem Grund ist es im Rahmen der erfindungsgemäßen Darreichungsform besonders vorteilhaft, diese dahingehend zu schützen, in dem man Antioxidantien, wie z.B. Gallussäurederivate, wie z.B. Propylgallat bzw. Salze der Gallussäure zusetzt. Auf diese Weise gelingt es, die flüssigen, wässrigen Darreichungsformen auch im weitestgehend neutralen Bereich stabil zu halten und eine entsprechende Lagerzeit bzw. Stabilität zu gewährleisten.

Mit Vorteil wird die flüssige, wässrige Darreichungsform wie schon erwähnt, durch die Zugabe von Lösungsmitteln bzw. Lösungsvermittlern erweitert, wie z.B. durch Polysorbate. Prinzipiell kann die Gruppe der Lösungsmittel bzw. -vermittler auf die meisten üblicherweise als pharmakologisch verträglich eingestuften Lösungsmittel bzw. -vermittler (Polysorbat, Natriumlaurylsülfat, Polyoxylstearate, Polyoxyloleylether, Polyoxyl-Rizinusöl) ausgedehnt werden, wobei auch einfache Alkohole wie Methanol, Propylenglycol, Glycerin, Polyethyleriglycol in Mengen zum Einsatz gelangen können, welche physiologisch bei sublingualer und nasaler Anwendung verträglich sind.

In besonders vorteilhafter Weise liegen erfindungsgemäß als Wirkstoff Buprenorphin, Naphazolin, Hydromorphon, Diphenhydramin Lidocain oder Nicotin jeweils in Form ihrer Salze, insbesondere als Hydrochloride, vor. Als Antihistaminika werden hierbei bevorzugt H1-Antihistaminika eingesetzt.

Die erfindungsgemäßen Zubereitungen erlauben es bei nasaler bzw. sublingualer Anwendung einen besonders raschen Wirkungseintritt bei gleichzeitig niedriger Dosierung sicherzustellen. Überraschenderweise hat sich auch gezeigt, dass die therapeutische Wirkungsdauer bezogen auf die Dosierung verlängert werden konnte, was nicht zuletzt durch die weitestgehend vollständige Resorption durch Diffusion durch Schleimhaut oder Mucosa erklärt werden kann.

Die Erfindung wird nachfolgend anhand der Zeichnung und von Ausführungsbeispielen näher erläutert. In der Zeichnung zeigt Fig.1 schematisch den Zeitraum, der für die Diffusion durch die Mucosa im Fall von Lidocain als Wirkstoff mit oder ohne Kaliumsorbat benötigt wird, und es ist aus der Zeichnung unmittelbar ersichtlich, dass bereits nach kurzer Zeit Lidocain mit Sorbat in 3 bis 4-facher Menge diffundiert als ohne Kaliumsorbat. Fig.2 veranschaulicht die Wirkungsintensität von Diphenhydramin gemessen an einem Versuchstier: Male C57BL/6 mice mit den Testmethoden: open field with infrared photocells (locomoto).

Bei den einzelnen Zubereitungen wurden verschiedene Gruppen von Wirkstoffen untersucht.
1) Hier wurde als Wirkstoff Lidocain Hydrochlorid, ein Wirkstoff aus der Gruppe der Antiarrhytmika als Nasenspray formliert. Die Dosiermenge wurde auf Sprühstöße von 50 bis 150 mg ausgelegt, wobei die Wirkstoffmenge pro Dosierung zwischen 0,25 mg und 6 mg je Sprühstoß gewählt wurde. Typische-Zusammensetzungen werden nachfolgend tabellarisch gelistet:
Beispiel 1 2 %-ige Lösung

| Stoffe | mg |
|---|---|
| Lidocain Hydrochlorid | 300 |
| Kaliumsorbat | 300 |
| Aqua Purificata | 14400 |
| Summe | 15000 |

Beispiel 2 4 %-ige Lösung

| Stoffe | mg |
|---|---|
| Lidocain Hydrochlorid | 600 |
| Kaliumsorbat | 600 |
| Aqua Purificata | 13800 |
| Summe | 15000 |

Beispiel 3 0,5 %-ige Lösung

| Stoffe | mg |
|---|---|
| Lidocain Hydrochlorid | 75 |
| Kaliumsorbat | 75 |
| Aqua Purificata | 14850 |
| Summe | 15000 |

2) Hier wurde ein Antihistaminikum, und im besonderen Diphenhydramin Hydrochlorid bzw. Naphazolin Hydrochlorid alleine oder gemeinsam als Nasenspray formuliert. Die Dosiermenge wurde wiederum auf Sprühstöße vom 50 bis 150 mg ausgelegt, wobei die Wirkstoffmenge pro Dosierung zwischen 25 µg und 300 µg je Sprühstoß gewählt wurde. Die typischen Zusammensetzungen für die einzelnen Lösungen werden nachfolgend wiederum tabellarisch wiedergegeben:
Beispiel 4 0,1% -ige Lösung

| Stoffe | mg |
|---|---|
| Diphenhydramin Hydrochlorid | 20 |
| Käliumsorbat | 20 |
| Natriumchlorid | 120 |
| Aqua Purificata | 19840 |
| Summe | 20000 |

Beispiel 5 0,2 %-ige Lösung

| Stoffe | mg |
|---|---|
| Diphenhydramin Hydrochlorid | 40 |
| Kaliumsorbat | 40 |
| Natriumchlorid | 100 |
| Aqua Purificata | 19820 |
| Summe | 20000 |

Beispiel 6 0,05 %-ige Lösung

| Stoffe | mg |
|---|---|
| Diphenhydramin Hydrochlorid | 10 |
| Kaliumsorbat | 10 |
| Natriumchlorid | 140 |
| Aqua Purificata | 19840 |
| Summe | 20000 |

Beispiel 7 0,1 %-ige Lösung

| Stoffe | mg |
|---|---|
| Diphenhydramin Hydrochlorid | 20 |
| Naphazolin Hydrochlorid | 20 |
| Kaliumsorbat | 40 |
| Natriumchlorid | 120 |
| Aqua Purificata | 19800 |
| Summe | 20000 |

Beispiel 8 0,2 %-ige Lösung

| Stoffe | mg |
|---|---|
| Diphenhydramin Hydrochlorid | 40 |
| Naphazolin Hydrochlorid | 40 |
| Kaliumsorbat | 80 |
| Natriumchlorid | 80 |
| Aqua Purificata | 19760 |
| Summe | 20000 |

Beispiel 9 0,05 %-ige Lösung

| Stoffe | mg |
|---|---|
| Diphenhydramin Hydrochlorid | 10 |
| Naphazolin Hydrochlorid | 10 |
| Kaliumsorbat | 20 |
| Natriumchlorid | 140 |
| Aqua Purificata | 19820 |
| Summe | 20000 |

3) Als Analgetikum der Gruppe Opioide wurde als Wirkstoff Buprenorphin Hydrochlorid wiederum als Nasenspray formuliert. Auch hier wurde die Dosiermenge wieder auf 50 bis 150 mg ausgelegt, wobei die Wirkstoffmenge je Dosierung zwischen 25 µg und 300 µg je Sprühstoß betrug. Getestete Zusammensetzungen werden in den nachfolgenden Beispielen 10, 11 und 12 wiederum tabellarisch gelistet:
Beispiel 10 0,1 %-ige Lösung

| Stoffe | mg |
|---|---|
| Buprenorphin Hydrochlorid | 20 |
| Kaliumsorbat | 20 |
| Propylgallat | 10 |
| 96 % Ethanol | 200 |
| Propylenglykol | 300 |
| Polysorbat 80 | 600 |
| Aqua Purificata | 18850 |
| Summe | 20000 |

Beispiel 11 0,2 %-ige Lösung

| Stoffe | mg |
|---|---|
| Buprenorphin Hydrochlorid | 40 |
| Kaliumsorbat | 40 |
| Propylgallat | 20 |
| 96 % Ethanol | 400 |
| Propylenglykol | 600 |
| Polysorbat 80 | 1200 |
| Aqua Purificata | 17700 |
| Summe | 20000 |

Beispiel 12 0,05 %-ige Lösung

| Stoffe | mg |
|---|---|
| Buprenorphin Hydrochlorid | 10 |
| Kaliumsorbat | 10 |
| Propylgallat | 5 |
| 96 % Ethanol | 100 |
| Propylenglykol | 150 |
| Polysorbat 80 | 300 |
| Aqua Purificata | 19425 |
| Summe | 20000 |

4) Aus der Gruppe der Analgetika bzw. Antidote, und insbesondere der Opioide, wurde wiederum Buprenorphin Hydrochlorid ausgewählt und diesmal als Bucalspray formuliert. Der Sprühstoß wurde wiederum mit 50 bis 150 mg dosiert, wobei die Wirkstoffmenge pro Dosierung 500 µg bis 6000 µg je Sprühstoß betrug. Die getesteten Zusammensetzungen sind in den Beispielen 13, 14 und 15 wiederum in ihrer Zusammensetzung tabellarisch identifiziert:
Beispiel 13 2,0 %-ige Lösung

| Stoffe | mg |
|---|---|
| Buprenorphin Hydrochlorid | 100 |
| Kaliumsorbat | 100 |
| Propylgallat | 20 |
| 96 % Ethanol | 1500 |
| Propylenglykol | 2795 |
| Cremophor | 250 |
| Menthol | 25 |
| Na-Saccharinat | 10 |
| Aqua Purificata | 200 |
| Summe | 5000 |

Beispiel 14 4,0 %-ige Lösung

| Stoffe | mg |
|---|---|
| Buprenorphin Hydrochlorid | 200 |
| Kaliumsorbat | 200 |
| Propylgallat | 40 |
| 96 % Ethanol | 1500 |
| Propylenglykol | 2540 |
| Cremophor | 250 |
| Menthol | 50 |
| Na-Saccharinat | 20 |
| Aqua Purificata | 200 |
| Summe | 5000 |

Beispiel 15 1,0 %-ige Lösung

| Stoffe | mg |
|---|---|
| Buprenorphin Hydrochlorid | 50 |
| Kaliumsorbat | 50 |
| Propylgallat | 10 |
| 96 % Ethanol | 1500 |
| Propylenglykol | 2917,5 |
| Cremophor | 250 |
| Menthol | 12,5 |
| Na-Saccharinat | 10 |
| Aqua Purificata | 200 |
| Summe | 5000 |

5) Hier wurde als Wirkstoff Nicotin, ein Wirkstoff aus der Gruppe der Rauchentwöhner als Bucalspray formuliert. Die Dosiermenge wurde auf Sprühstöße von 40 bis 160 mg ausgelegt, wobei die Wirkstoffmenge pro Dosierung zwischen 0,2 mg und 3,2 mg je Sprühstoß gewählt wurde. Typische Zusammensetzungen werden nachfolgend tabellarisch gelistet:
Beispiel 16 1 %-ige Lösung

| Stoffe | mg |
|---|---|
| Nicotin | 500 |
| Kaliumsorbat | 500 |
| Ascorbinsäure | 500 |
| Natriumsaccharinat | 100 |
| Orangenaroma | 100 |
| Wasser | 48300 |
| Summe | 50000 |

Beispiel 17 2 %-ige Lösung

| Stoffe | mg |
|---|---|
| Nicotin | 1000 |
| Kaliumsorbat | 1000 |
| Ascorbinsäure | 1000 |
| Natriumsaccharinat | 100 |
| Orangenaroma | 100 |
| Wasser | 46800 |
| Summe | 50000 |

Beispiel 18 0,5 %-ige Lösung

| Stoffe | mg |
|---|---|
| Nicotin | 250 |
| Kaliumsorbat | 250 |
| Ascorbinsäure | 250 |
| Natriumsaccharinat | 100 |
| Orangenaroma | 100 |
| Wasser | 49050 |
| Summe | 50000 |

## Patentansprüche

1. Flüssige, wässrige Darreichungsform für sublinguale oder nasale Anwendung von Aminogruppen aufweisenden Wirkstoffen, gewählt aus der Gruppe enthaltend Analgetika, insbesondere Opiate, Lokalanästhetika, Antihistaminika oder Nicotin, **dadurch gekennzeichnet, daß** der Wirkstoff als pharmakologisch unbedenkliches Salz eingesetzt ist und mit K-Sorbat zur raschen Diffusion in wenigstens äquimolarer Menge in der Flüssigkeit vorliegt.

2. Flüssige, wäßrige Darreichungsform nach Anspruch 1, **dadurch gekennzeichnet, daß** die Lösung Antioxidantien enthält.

3. Flüssige, wäßrige Darreichungsform nach Anspruch 1 oder 2, **dadurch gekennzeichnet, daß** als Antioxidantien Gallussäurederivate, wie z.B. Propylgallat bzw. Salze der Gallussäure eingesetzt sind.

4. Flüssige, wäßrige Darreichungsform nach Anspruch 1, 2 oder 3, **dadurch gekennzeichnet, daß** die Lösung Lösungsvermittler, wie z.B. Polysorbate oder Polyethylenoxide enthält.

5. Flüssige, wäßrige Darreichungsform nach einem der Ansprüche 1 bis 4, **dadurch gekennzeichnet, daß** als Lösungsvermittler Polysorbat, Natriumlaurylsulfat, Polyoxylstearate, Polyoxyloleylether und Polyoxyl-Rizinusöl eingesetzt sind.

6. Flüssige, wäßrige Darreichungsform nach einem der Ansprüche 1 bis 5, **dadurch gekennzeichnet, daß** als Wirkstoff Buprenorphin, Naphazolin, Hydromorphon, Diphenhydramin oder Lidocain, jeweils in Form ihrer Salze, insbesondere als Hydrochloride, vorliegen.

7. Flüssige, wäßrige Darreichungsform nach einem der Ansprüche 1 bis 6, **dadurch gekennzeichnet, daß** als Antihistaminika H1-Antihistaminika eingesetzt sind.

## Claims

1. A liquid, aqueous dosage form for sublingual or nasal applications of active ingredients containing amino groups and selected from the group consisting of analgesics, in particular opiates, local anaesthetics, antihistamines or nicotine, **characterized in that** the active ingredient is used as a pharmacologically safe salt and is contained in the liquid together with K-sorbate for rapid diffusion in at least equimolar amount.

2. A liquid, aqueous dosage form according to claim 1, **characterized in that** the solution contains antioxidants.

3. A liquid, aqueous dosage form according to claim 1 or 2, **characterized in that** gallic acid derivatives such as, e.g., propyl gallate or salts of gallic acid are used as antioxidants.

4. A liquid, aqueous dosage form according to claim 1, 2 or 3, **characterized in that** the solution contains solubilizers such as, e.g., polysorbate or polyethylene oxide.

5. A liquid, aqueous dosage form according to any one of claims 1 to 4, **characterized in that** polysorbate, sodium lauryl sulfate, polyoxyl stearates, polyoxylene ethyl ether and polyoxyl castor oil are used as solubilizers.

6. A liquid, aqueous dosage form according to any one of claims 1 to 5, **characterized in that** buprenorphine, naphazoline, hydromorphone, diphenhydramine or lidocaine are contained as active ingredients, each in their salt forms, in particular as hydrochlorides.

7. A liquid, aqueous dosage form according to any one of claims 1 to 6, **characterized in that** H1-antihiatamines are used as antihistamines.

## Revendications

1. Forme galénique aqueuse liquide pour l'application sublinguale ou nasale de principes actifs présentant des groupes amino, choisis dans le groupe contenant des analgésiques, en particulier des opiacés, des anesthésiques locaux, des antihistaminiques ou de la nicotine, **caractérisée en ce que** le principe actif est utilisé sous forme de sel pharmacologiquement inoffensif et se présente avec du sorbate de potassium pour la diffusion rapide en au moins une quantité équimolaire dans le liquide.

2. Forme galénique aqueuse liquide selon la revendication 1, **caractérisée en ce que** la solution contient des antioxydants.

3. Forme galénique aqueuse liquide selon la revendication 1 ou 2, **caractérisée en ce que** l'on utilise comme antioxydants, des dérivés d'acide gallique, tels que par exemple le propylgallate cu des sels d'acide gallique.

4. Forme galénique aqueuse liquide selon la revendication 1, 2 ou 3, **caractérisée en ce que** la solution contient des agents solubilisants, tels que par exemple, des polysorbates ou des oxydes de polyéthylène.

5. Forme galénique aqueuse liquide selon l'une quelconque des revendications 1 à 4, **caractérisée en ce que** l'on utilise comme agent solubilisant, du polysorbate, du laurylsulfate de sodium, du stéarate de polyoxyle, de l'oléyléther de polyoxyle et de l'huile de ricin polyoxyle.

6. Forme galénique aqueuse liquide selon l'une quelconque des revendications 1 à 5, **caractérisée en ce que** l'on utilise comme principe actif, de la buprénorphine, de la naphazoline, de l'hydromorphone, de la diphénhydramine ou de la lidocaïne, respectivement sous la forme de leurs sels, en particulier sous forme de chlorhydrate.

7. Forme galénique aqueuse liquide selon l'une quelconque des revendications 1 à 6, **caractérisée en ce que** l'on utilise comme antihistaminiques, des antihistaminiques H1.
